(19) Europäisches Patentamt | European Patent Office | Office européen des brevets

(11) **EP 3 007 713 B1**

(12) **EUROPEAN PATENT SPECIFICATION**

(45) Date of publication and mention
of the grant of the patent:
**23.10.2019 Bulletin 2019/43**

(21) Application number: **14748292.1**

(22) Date of filing: **11.06.2014**

(51) Int Cl.:
*A61K 36/73* (2006.01)      *A61P 17/00* (2006.01)
*A61K 8/36* (2006.01)      *A61K 8/49* (2006.01)
*A61Q 19/06* (2006.01)      *A61Q 19/08* (2006.01)
*A61K 31/192* (2006.01)      *A61K 31/352* (2006.01)
*A61K 8/92* (2006.01)
*A61K 8/365* (2006.01)

(86) International application number:
**PCT/IT2014/000160**

(87) International publication number:
**WO 2014/199408 (18.12.2014 Gazette 2014/51)**

(54) **OILY EXTRACT OF PLANTS OF RUBUS SPECIES AND USES THEREOF IN MEDICAL AND COSMETIC FIELDS**

ÖLIGER PFLANZENEXTRAKT DER SPEZIES RUBUS UND VERWENDUNGEN DAVON IM MEDIZINISCHEN UND KOSMETISCHEN BEREICH

EXTRAIT HUILEUX DE PLANTES D'ESPÈCES DE RUBUS ET SES UTILISATIONS DANS LES DOMAINES MÉDICAL ET COSMÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.06.2013 IT RM20130337**

(43) Date of publication of application:
**20.04.2016 Bulletin 2016/16**

(73) Proprietor: **Medicina Vegetale Tradizionale Group S.r.l.**
**09010 Pula (CA) (IT)**

(72) Inventors:
• **ZARU, Marco**
  **09010 Pula (CA) (IT)**
• **TRONCI, Maria Bonaria**
  **09010 Pula (CA) (IT)**
• **BIGGIO, Giovanni**
  **09010 Pula (CA) (IT)**
• **BIGGIO, Teobaldo**
  **09010 Pula (CA) (IT)**
• **PIRAS, Carmelo**
  **09010 Pula (CA) (IT)**
• **BOI, Bonarina**
  **09010 Pula (CA) (IT)**
• **BOI, Ignazia**
  **09010 Pula (CA) (IT)**
• **BOI, Maria**
  **09010 Pula (CA) (IT)**
• **BOI, Stefanina**
  **09010 Pula (CA) (IT)**
• **BOI, Carlo**
  **09010 Pula (CA) (IT)**
• **BOI, Elena**
  **09010 Pula (CA) (IT)**

(74) Representative: **Gitto, Serena et al**
**Barzanò & Zanardo Roma S.p.A.**
**Via Piemonte, 26**
**00187 Roma (IT)**

(56) References cited:
**IT-A1- CA 930 003**

• **Anonymous: "Blackberry", , 28 January 2013 (2013-01-28), XP002718101, Retrieved from the Internet: URL:http://www.activenaturalsinstitute.com /library_blackberry [retrieved on 2013-12-17]**

- ESRA SARIBURUN ET AL: "Phenolic Content and Antioxidant Activity of Raspberry and Blackberry Cultivars", JOURNAL OF FOOD SCIENCE, vol. 75, no. 4, 1 May 2010 (2010-05-01), pages C328-C335, XP055093962, ISSN: 0022-1147, DOI: 10.1111/j.1750-3841.2010.01571.x
- J. M. Calderon-Montano ET AL: "A Review on the Dietary Flavonoid Kaempferol", Mini-Reviews in Medicinal Chemistry, 1 April 2011 (2011-04-01), pages 298-344, XP055093966, DOI: 10.2174/138955711795305335 Retrieved from the Internet: URL:http://www.eurekaselect.com/openurl/content.php?genre=article&issn=13895575&volume=11&issue=4&spage=298 [retrieved on 2013-12-17]
- T SIRIWOHARN ET AL: "JFS: Food Chemistry and Toxicology Polyphenolic Composition of Marion and Evergreen Blackberries", 2004-JOURNAL OF FOOD SCIENCE FCT233, vol. 69, no. 4, 15 April 2004 (2004-04-15) , pages 233-240, XP055093980,
- HUMMER K E: "Rubus Pharmacology: Antiquity to the Present", HORTSCIENCE, AMERICAN SOCIETY OF HORTICULTURAL SCIENCE, ALEXANDRIA, VA, US, vol. 45, no. 11, 1 November 2010 (2010-11-01), pages 1587-1591, XP009144579, ISSN: 0018-5345 [retrieved on 2010-11-01]
- K. HAMMER ET AL: "Dynamics of Rubus ulmifolius Schott var. anoplothyrsus Sudre and other cultivated blackberries in Italy", GENETIC RESOURCES AND CROP EVOLUTION, vol. 51, no. 3, 1 May 2004 (2004-05-01), pages 237-239, XP055093942, ISSN: 0925-9864, DOI: 10.1023/B:GRES.0000024026.26655.d7
- M.A. MARQUINA ET AL: "Hyaluronidase inhibitory activity from the polyphenols in the fruit of blackberry (Rubus fruticosus B.)", FITOTERAPIA, vol. 73, no. 7-8, 1 December 2002 (2002-12-01), pages 727-729, XP055093910, ISSN: 0367-326X, DOI: 10.1016/S0367-326X(02)00222-8
- MARIA LETIZIA MANCA ET AL: "A new technological approach to improve the efficacy of a traditional herbal medicinal product in wound healing", INDUSTRIAL CROPS AND PRODUCTS., vol. 63, 7 November 2014 (2014-11-07), pages 71-78, XP055446060, NL ISSN: 0926-6690, DOI: 10.1016/j.indcrop.2014.10.049

**Description**

The present invention relates to an oily extract of plants of *Rubus* species and uses thereof in medical and cosmetic fields. In more detail, the invention relates to the use of an oily extract of plants of *Rubus* species and the topical treatment of cutaneous and mucosal pathologies and skin lesions. The invention further concerns a method for obtaining such extract through extraction in oil at room temperature.

As is well known, many plant-extract based formulations are currently used for treating skin lesions of various extensions and natures, thanks to their dermoprotective action and cicatrisation, anti-inflammatory, antibacterial, anti-dehydrating and soothing properties. For example, propolis-based creams are currently commercialised since they are able to stimulate tissue regeneration in the event of wounds or sores and promote the absorption of vitamin C, which is important for the synthesis of collagen.

In 2005, Maha F. El Goweni et AL, 2005, demonstrated that quercetin bioflavonoid has both preventive and curative effects in the treatment of hypertrophic scars. Subsequently, thanks to the study performed by Ipek Süntar et AL., 2009, "Wound Healing Activity of Rubus Sanctus Schreber (Rosacee): Preclinical study in Animal models*"*, it was suggested how extracts obtained from aerial parts of plants belonging to the *Rubus Sanctus* species can lead to the healing of wounds. However, such extracts were obtained through extraction with a solvent (n-hexane, methanol, chloroform, ethyl acetate, etc.) which, as is well known, implies a series of problems both in terms of toxicity and cost. For example, the publications of Esra Sariburun et al. (Esra Sariburun et al., Journal of Food Science, vol. 75, no. 4, 2010, pages C328-335) and Siriwoharn et al. (Siriwoharn et al., Journal of Food Science, vol. 69, no.4, 2004, pages 233-240) are known, which describe extraction processes of the *Rubus* species through the use of water/methanol hydroalcoholic solution or organic solvents such as chloroform or dichloromethane. The extracts obtained through the extraction methods mentioned above can therefore contain residuals of organic solvents that are harmful to health.

Furthermore, it must be considered that the use of various solvents causes particular divisions of the components extractable from the plant based on the different affinity of each component to the solvent used. Therefore, the use of organic solvents or various organic solvents in sequence, for example, two or three organic solvents in sequence, could result in a final extract in which not all the active substances or the adjuvant substances of the phytocomplex are present.

Patent IT 0001265649 described an extraction process for Rubus in oil, said process being characterised by the use of a high temperature and the use of fresh buds. However, in the process conditions of the prior art, only a minimal negligible percentage of components of a polyphenolic nature was yielded to the solvent.

In light of the above, the need to be able to make use of a new method to overcome the drawbacks of known methods appears clear.

The solution according to the present invention, which aims to provide an oily extract of *Rubus* characterised by a high polyphenol content, suitable for the treatment of skin and mucosal lesions, falls within this context.

Furthermore, a method was found for obtaining said extract characterised in that the extraction is a cold oil extraction starting from a dry plant sample.

The extract that forms the subject matter of the present invention allows notably more marked, effective and rapid beneficial therapeutic effects to be obtained in comparison with existing antibiotic and/or cortisone-based dermatological and cicatrisation therapies. In fact, said extract has marked astringent, antiphlogistic, antiseptic, haemostatic and soothing activity, which is difficult to encompass in a single solution traceable to products already existing on the market.

The action of the extract according to the present invention is manifested on the surfaces of the skin and mucosa through the formation of a protective cuticle, due to the precipitation and transformation of tissue proteins, against chemical, bacterial, mechanical and irritative agents in general. This leads to a reduction in the secretory processes with more intensive removal of water from the tissues the more said tissues are imbibited, as actually happens in inflammatory processes. In burns in particular, the extract according to the present invention soothes pain, prevents losses of serum and protects the wound from bacterial invasion.

The extract further promotes excellent tissue cicatrisation with complete restitution of the damaged tissues and the absence of scarring retraction, giving the newly formed tissue excellent elasticity. Due to these special characteristics, the subject matter of the present invention proposes an innovative solution in comparison with the products traditionally used in such pathologies.

Furthermore, unlike the Rubus extracts obtained through the use of organic solvents, the extract according to the present invention is obtained through the use of oil, in particular natural vegetable oil. The extraction process according to the present invention does not envisage the use of any organic solvents, either lipophilic or hydrophilic. Therefore, the extract according to the present invention does not contain solvent residuals that are harmful to health.

A further advantage of the extract according to the present invention is represented by the fact that the extract does not contain products of the degradation of the thermolabile components of the plant since the extraction process according to the present invention is conducted cold. In fact, the degradation of the active substances would lead to a loss or reduction of the anti-oxidising power of the extract.

Based on the analytical results reported in the examples of the present description, it can also be observed that the extract according to the present invention contains significant quantities of all the active polyphenolic components of the phytocomplex such as, for example, caffeic acid, quercetin, rhamnetin and Kaempferol.

Clinical evidence, lacking in controlled studies, shows that the clinical results obtained through the use of the extract according to the present invention cannot be correlated solely and exclusively to the quantity and/or type of antioxidant contained in the extract and deriving from the plant species subjected to extraction. The clinical results, lacking in controlled studies, could also be due to a series of synergies installed between the phytocomplex contained in the extract and the oil used for extraction as well as any further synergies with the rest of the components of the pharmaceutical form containing the extract.

Unlike the extraction process reported in Italian patent IT 0001265649 mentioned above, characterised by a high temperature and the use of fresh buds, the preparation method of the extract according to the invention, which uses dry drug and low temperatures, allows a very high concentration of the polyphenolic fraction to be obtained, rich in particular in flavones, flavonols and flavonoids, which certainly includes a series of substances with activities and potential in the dermatological field. As shown in example 2 of the present patent application, the content of polyphenols in the extract obtained according to the process of the present invention was compared with that obtained through the process described by Italian patent IT 0001265649. The phenolic component of the extract according to the present invention was much higher than that of the patent mentioned above wherein said phenolic component is almost non-existent to the extent that it was not detected by the analytical instrument used.

Furthermore, it is possible that the extraction conditions used in the prior art are not able to extract all the active substances such as, for example, caffeic acid, rhamnetin, quercetin and kaempferol. In the extract according to the present invention it is possible to clearly detect the presence of significant and effective quantities of the polyphenolic complex comprising caffeic acid, rhamnetin, quercetin and Kaempferol. In particular, the extract according to the present invention contains Caffeic Acid at the concentration of at least 0.2% (w/w), Rhamnetin at the concentration of at least 0.3% (w/w), Quercetin at the concentration of at least 0.3% (w/w) and Kaempferol at the concentration of at least 0.2% (w/w).

Furthermore, it has been observed that, for the same content of extract, the preparation according to the present invention is more effective in comparison with the composition of the prior art described in Italian patent IT0001265649 for the treatment of skin and mucosal disorders or pathologies showing a more marked action promoting cutaneous regeneration and quicker healing.

Finally, comparative studies with creams available on the market and used for the same use as the extract according to the present invention show much greater efficacy of the extract according to the invention.

Hence, the specific object of the present invention is an oily extract of plants of *Rubus* species, said extract being obtained by cold oil extraction starting from dry plant samples and comprising the polyphenolic phytocomplex of said plants, wherein said polyphenolic phytocomplex comprises caffeic acid, rhamnetin, quercetin, kaempferol and wherein said dry plant samples are dried buds. In particular, the extract according to the present invention may contain Caffeic Acid at the concentration of at least 0.2% (w/w), Rhamnetin at the concentration of at least 0.3% (w/w), Quercetin at the concentration of at least 0.3% (w/w) and Kaempferol at the concentration of at least 0.2% (w/w), wherein said percentages are weight percentages of each compound with respect to the weight of the extract. In fact, the extract according to the present invention contains flavonoids such as rhamnetin, flavonols comprising quercetin, kaempferol, flavanonols, such as 3-hydroxyflavanones or 2,3-dihydroflavonols. The preparation process of the extract according to the present invention enables the phytocomplex of the plant to be extracted wherein the active ingredients are present without the need for intermediate processes that would reduce the synergies present in the phytocomplex itself and consequently in the finished pharmaceutical form.

Preferably, the plants of the *Rubus* species are chosen from the group consisting of *Rubus Ulmifolius, Rubus Idaeus, Rubus caesius, Rubus saxatillis,* from the group *Rubus Ulmifolius* preferably *Rubus ulmifolius frutticosus schott.*

Furthermore, according to the invention, the oil is chosen from the group consisting of vegetable oils with a marked polyunsaturated fatty acid component chosen from the group consisting of almond, jojoba, sunflower, virgin olive and extra virgin olive oils, preferably virgin olive and extra virgin olive oils. Said vegetable oils are characterised by a marked polyunsaturated fatty acid component such as, for example, linolenic, gamma linolenic.

The present invention further relates to a pharmaceutical composition comprising or consisting of an oily extract of plants of *Rubus,* as defined above, as an active ingredient, along with one or more pharmaceutically acceptable excipients and/or coadjuvants. Preferably, the pharmaceutical composition according to the present invention is a pharmaceutical composition for topical use.

A further aspect of the present invention is a process for the preparation of an oily extract of plants of *Rubus,* said process being characterised in that the extraction takes place through the incubation in oil of dried parts of plants of the *Rubus* species at a temperature that varies from 18 to 32 °C, preferably from 22 to 28°C, more preferably at 25°C, for an incubation time of 5-15 days, wherein the dried parts of plants are dried buds. Any part of the plant can be used in the extraction, however, the leaves and buds are preferable, wherein the buds (young shoots) are preferred.

In particular, in said process the *Rubus* species are chosen from the group consisting of *Rubus Ulmifolius, Rubus Idaeus, Rubus caesius, Rubus saxatillis,* from the group *Rubus Ulmifolius* preferably *Rubus ulmifolius frutticosus schott.*

Preferably, in said process, the oil is chosen from the group consisting of vegetable oils with a marked polyunsaturated fatty acid component chosen from the group consisting of almond, jojoba, sunflower, virgin olive and extra virgin olive oils, preferably virgin olive and extra virgin olive oils. Said vegetable oils being characterised by a marked polyunsaturated fatty acid component such as, for example, linolenic, gamma linolenic.

Furthermore, according to the present invention, in the aforementioned process, the plants of the Rubus species are harvested in the spring-summer seasons.

A further aspect of the present invention is an extract or a pharmaceutical composition as previously defined for use in the treatment of skin or mucosal disorders or pathologies. The skin or mucosal disorders or pathologies include for example burns, grazes, abrasions, rhagades, varicose ulcers, radiodermatitis, bedsores, torpid sores, light haemorrhages, nose bleeds, traumatic wounds, erythrodermic states, eczema, chilblains, dyshidrosis, itching, diabetic itching, insect bites, herpetic and erythematous lesions, dermatitis, for example psoriasis, atopic dermatitis, dermatosis, acne rosacea and vulgaris, folliculitis and furunculosis, post-laser erythema, nappy rash and sunrash, mouth ulcers, Lichen Planus, preferably burns and as a coadjuvant in the alteration of surgical wounds.

The extract and the pharmaceutical composition according to the present invention may be used in the medical and veterinary field, i.e. for treating both humans and animals.

Said extract and/or said composition can be applied both directly to the injured part of the body to be treated, for some hours, once a day, or by occlusion with bandages, gauze and similar materials for 30-60 minutes, 2-3 times a day.

In a preferred embodiment, the pharmaceutical composition according to the present invention comprises the following ingredients in the quantities listed below: sodium benzoate (as a preserving antimicrobial agent) 1 g, butylated hydroxytoluene (BHT) (as a preserving antioxidising agent) 0.1 g, lanolin from 120 to 350 g, preferably from 200 to 300g, even more preferably 250 g, wax from 50 to 150 g, preferably from 65 to 120, even more preferably 80 g, water from 75 to 350, preferably from 120 to 250, even more preferably 219 g, wherein the quantity of RUB oily extract is from 100 to 800, preferably from 200 to 500, more preferably 450 g.

The present invention further concerns a cosmetic composition comprising the extract as defined above. Furthermore, the present invention regards the use of the extract according to the invention and the cosmetic composition defined above as a skin cosmetic, for example, antiageing, antioxidising or against skin imperfections such as, for example, stains, scars, stretch marks and cellulite.

The present invention will now be described, according to its preferred embodiments, with particular reference to the figures of the attached drawings, wherein:

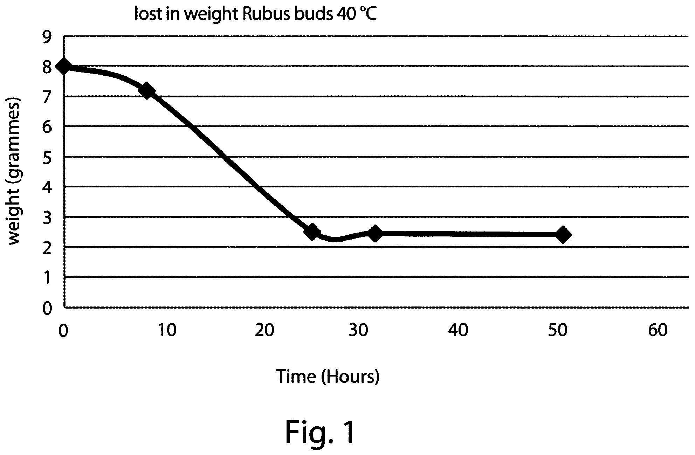

Figure 1 shows the weight variation of buds in relation to the period of time in the stove at 40°C;

Figure 2 shows the chromatogram obtained with a dried OLEO-RUB sample (obtained with extract prepared using the dry drug);

Figure 3 shows the chromatogram obtained with a sample prepared having a higher concentration of extract, keeping the analytical conditions unaltered;

Figure 4 shows a chromatogram obtained from a fresh sample (fresh drug);

Figure 5 shows a chromatogram obtained from a dry sample (dry drug);

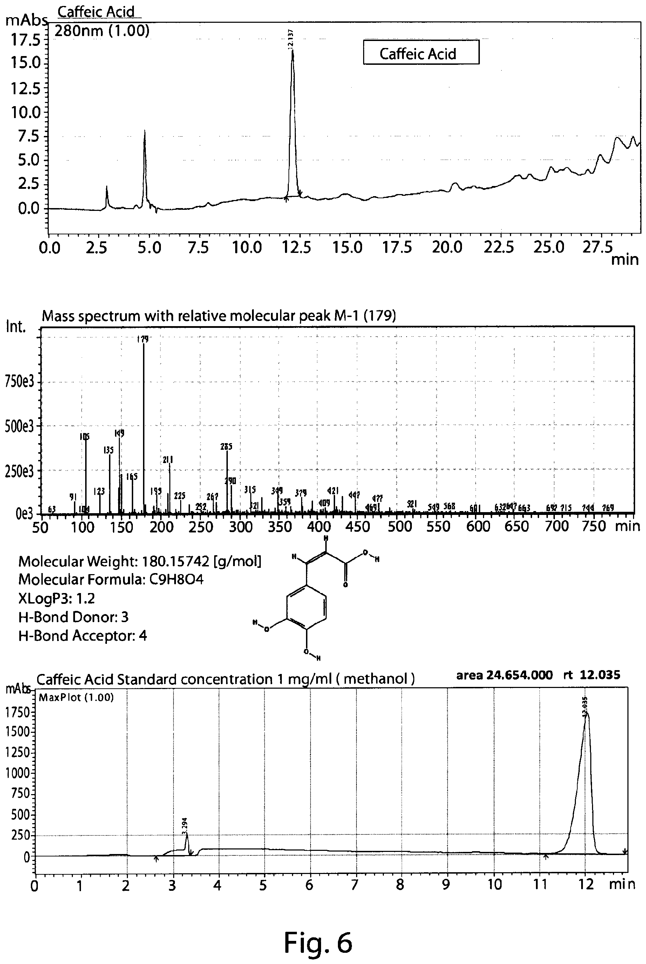

Figure 6 shows an HPLC chromatogram of a first fractionation of a sample subjected to five fractionations; a mass spectrum relative to the same first fractionation; a chromatogram obtained with a standard of caffeic acid at the concentration of 1 mg/ml (methanol);

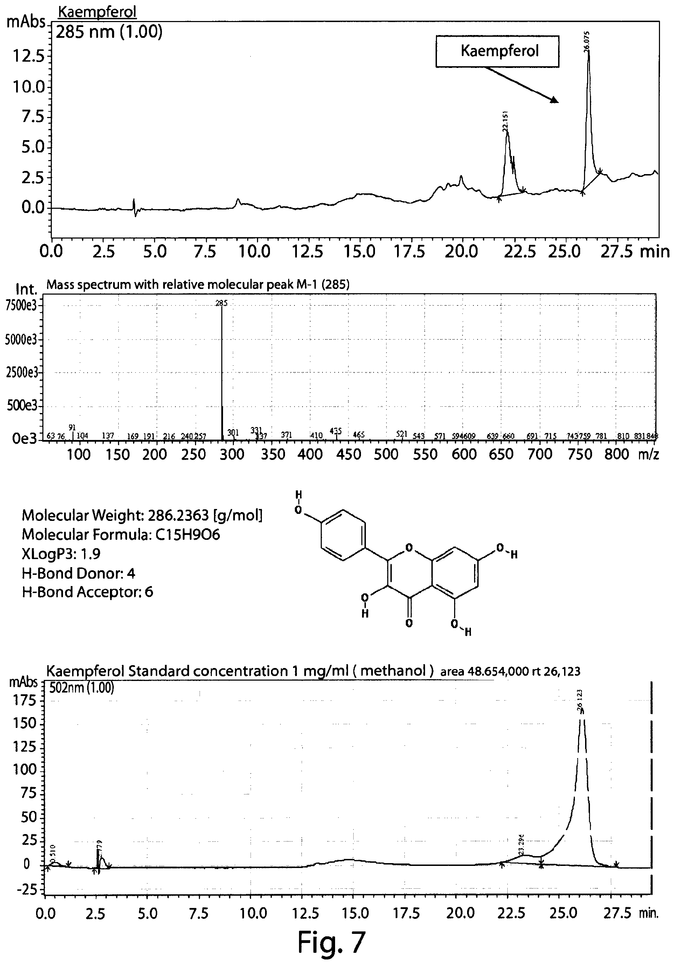

Figure 7 shows an HPLC chromatogram of a second fractionation of a sample subjected to five fractionations; a mass spectrum relative to the same second fractionation; a chromatogram obtained with a standard of kaempferol at the concentration of 1 mg/ml (methanol);

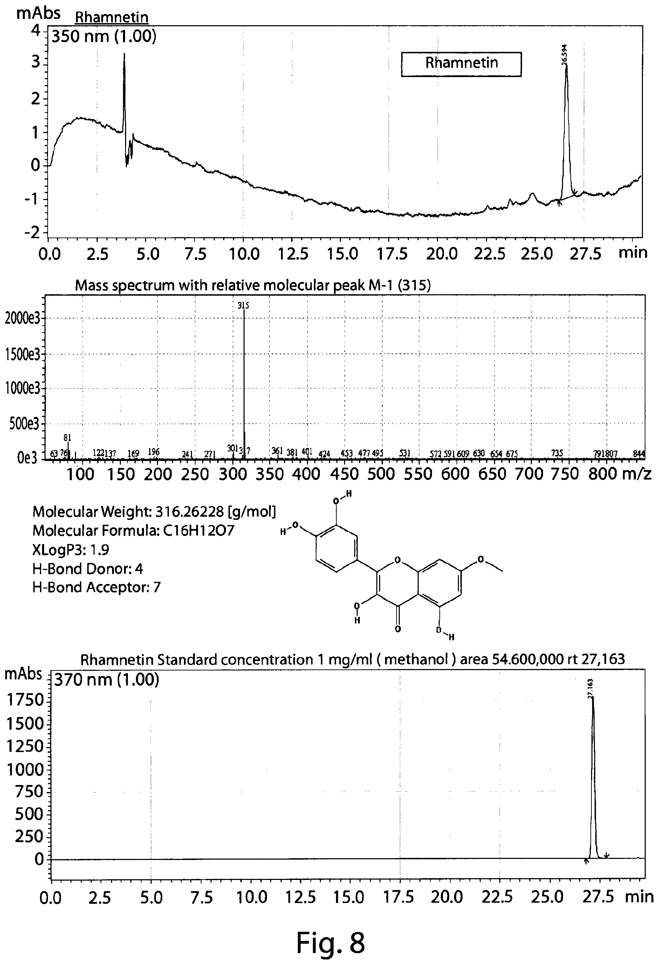

Figure 8 shows an HPLC chromatogram of a third fractionation of a sample subjected to five fractionations; a mass spectrum relative to the same third fractionation; a chromatogram obtained with a standard of rhamnetin at the concentration of 1 mg/ml (methanol);

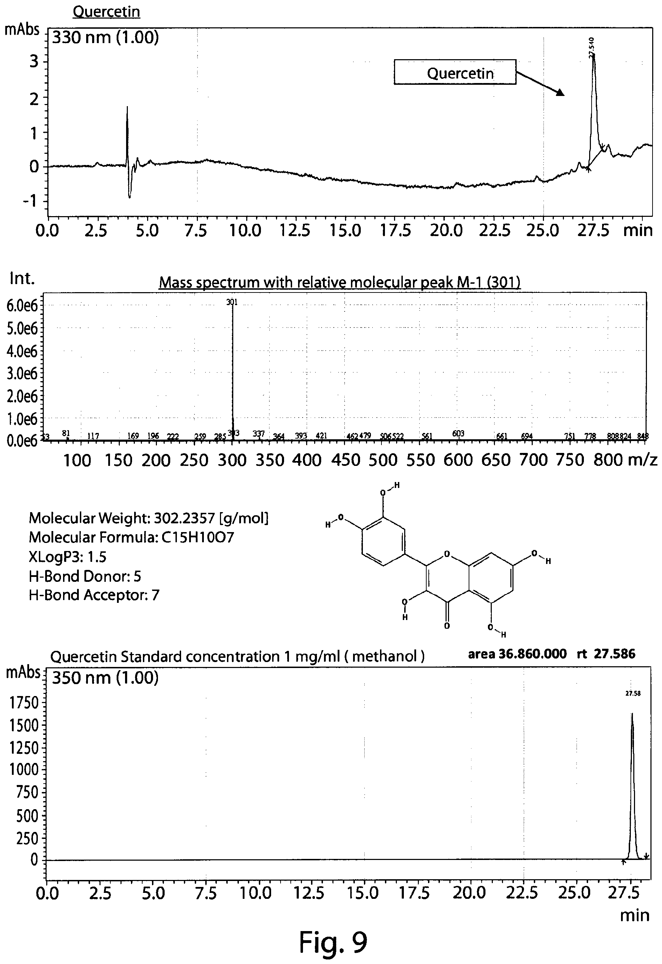

Figure 9 shows an HPLC chromatogram of a fourth fractionation of a sample subjected to five fractionations; a mass spectrum relative to the same fourth fractionation; a chromatogram obtained with a standard of quercetin at the concentration of 1 mg/ml (methanol);

Figure 10 shows an HPLC chromatogram of a fifth fractionation of a sample subjected to five fractionations; a mass spectrum relative to the same fifth fractionation;

Figure 11 shows the trend of the healing process of the wound at different times from the burn in a rat treated chronically with physiological (saline) solution only, with the commercial product Connettivina and with the cream according to the invention (MVT Skin Care);

Figure 12 shows the modifications of the area of the wound according to the time since the burn;

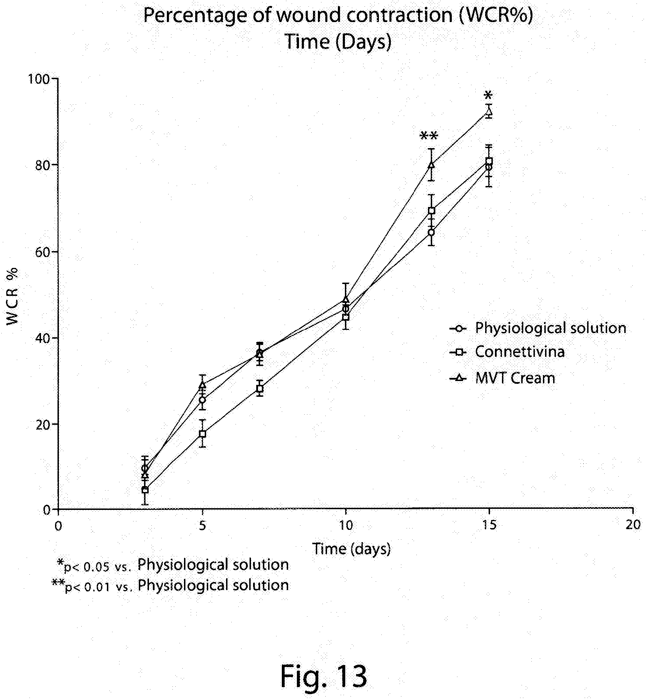

Figure 13 shows the modifications of the WCR% values according to the time since the burn;

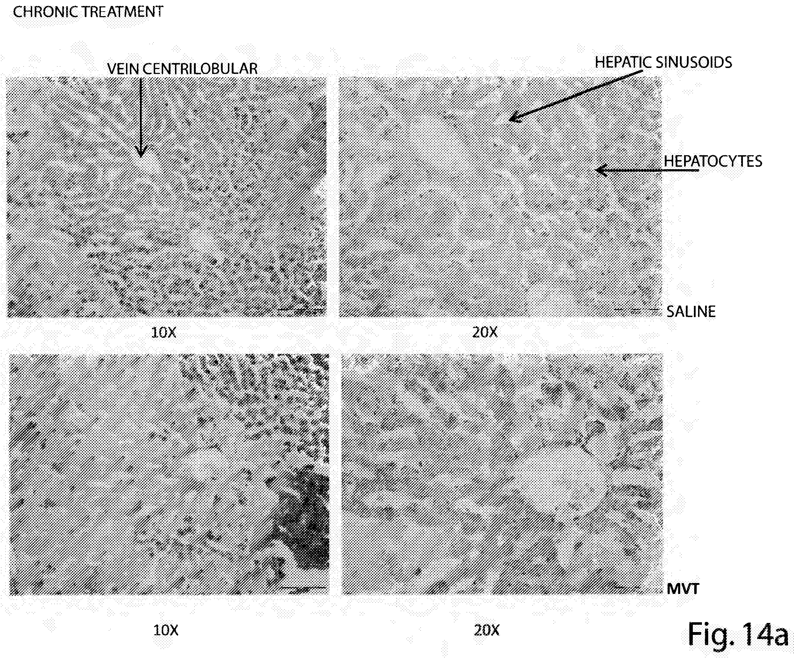

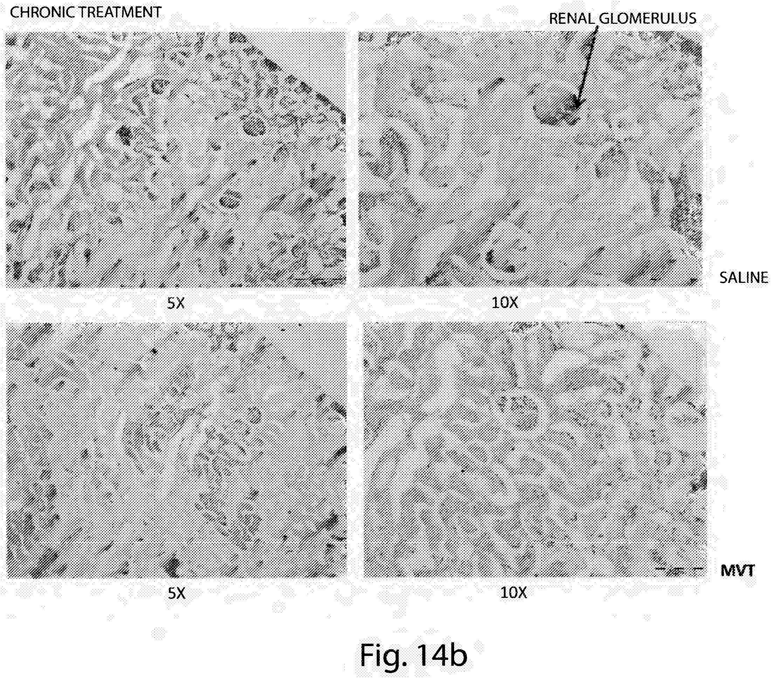

Figure 14 shows: Figure 14 a) shows micrographs of hepatic tissue coloured with haematoxylin and eosin, from a rat treated chronically with physiological (saline) solution only and from a rat treated chronically with the cream according to the invention (MVT Skin Care); the images were acquired with a 10X objective (images on the left) and a 20X objective (images on the right); Figure 14 b) shows micrographs of renal tissue coloured with haematoxylin and eosin, from a rat treated chronically with physiological (saline) solution only and from a rat treated chronically with the cream according to the invention (MVT Skin Care); the images were acquired with a 5X objective (images on the left) and a 10X objective (images on the right);

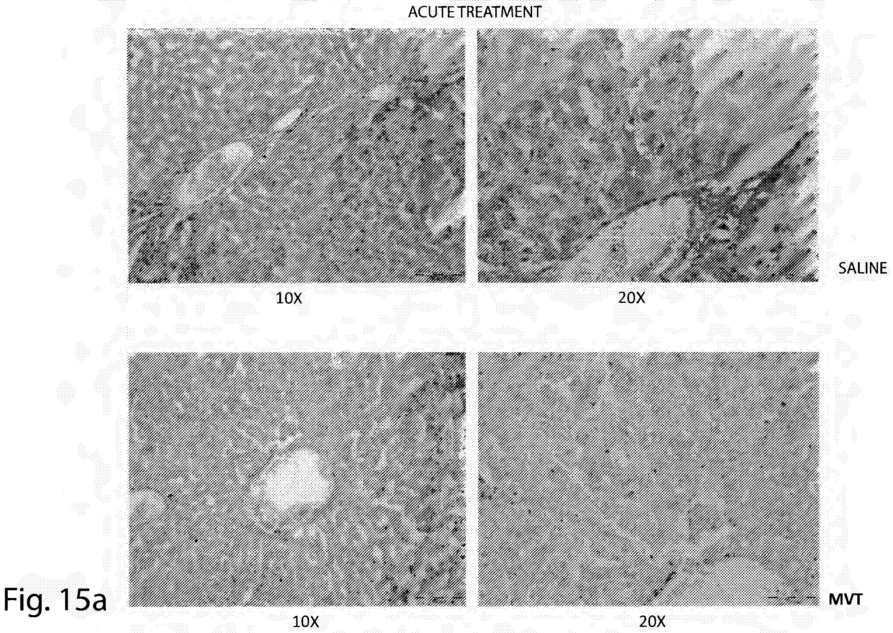

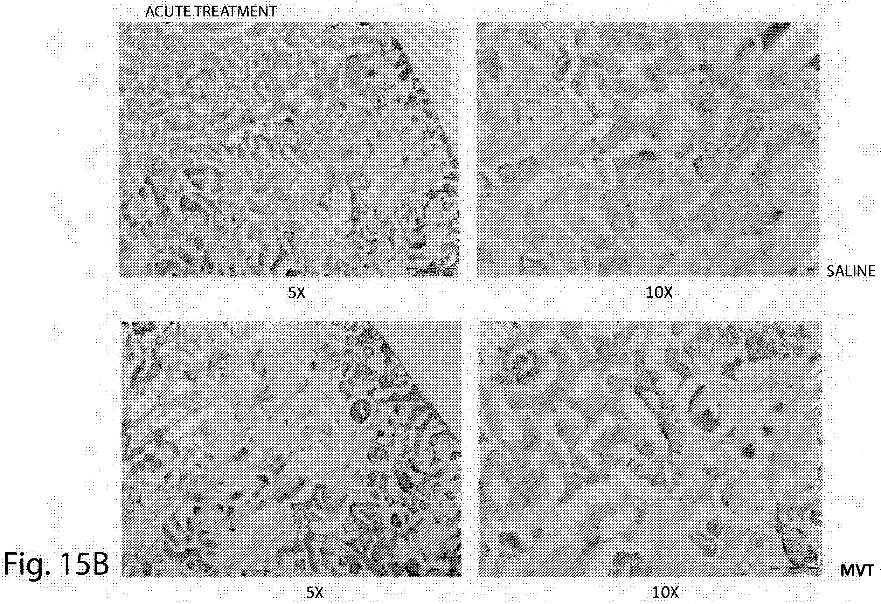

Figure 15 shows: Figure 15a) shows micrographs of hepatic tissue coloured with haematoxylin and eosin, from a rat treated acutely with physiological (saline) solution only and from a rat treated acutely with the cream MVT Skin Care; the images were acquired with a 10X objective (images on the left) and a 20X objective (images on the right); Figure 15 b) shows micrographs of renal tissue coloured with haematoxylin and eosin, from a rat treated acutely with physiological (saline) solution only and from a rat treated chronically with the cream according to the invention (MVT Skin Care); the images were acquired with a 1X objective (images on the left) and a 10X objective (images on the right).

Figure 16 shows the results of the HPLC analysis conducted on the extract obtained hot according to the process described in Italian patent IT 0001265649.

Figure 17 shows the results of the HPLC analysis conducted on the extract obtained according to the process described in the present invention.

The present invention will now be described, with particular reference to some specific embodiments.

## EXAMPLE 1

**PART 1 Formulation and Characterisation**

**Drying**

Buds of Rubus Ulmifolius are inserted into a ventilated stove at 40 $\pm$ 5°C for a minimum of 24 hours and a maximum of 48 hours, preferably 35 hours.

**Extraction**

(OLEO-RUB.ESS sample)(i.e. extract prepared using dry drug)

7.5 g (minimum 4 - maximum 10 days) of *Rubus Ulmifolius* buds dried in the stove, are placed in 92.5 g (minimum 75 g - maximum 110 g) of refined olive oil (official pharmacopoeia). The drug remains in contact with the oil for 10 days (minimum 5 - maximum 15 days) at room temperature, indicatively 25°C (minimum 18°C - maximum 32°C). After the preestablished time, the sample is filtered and appropriately stored.

**Quality investigation OLEO-RUB.ESS**

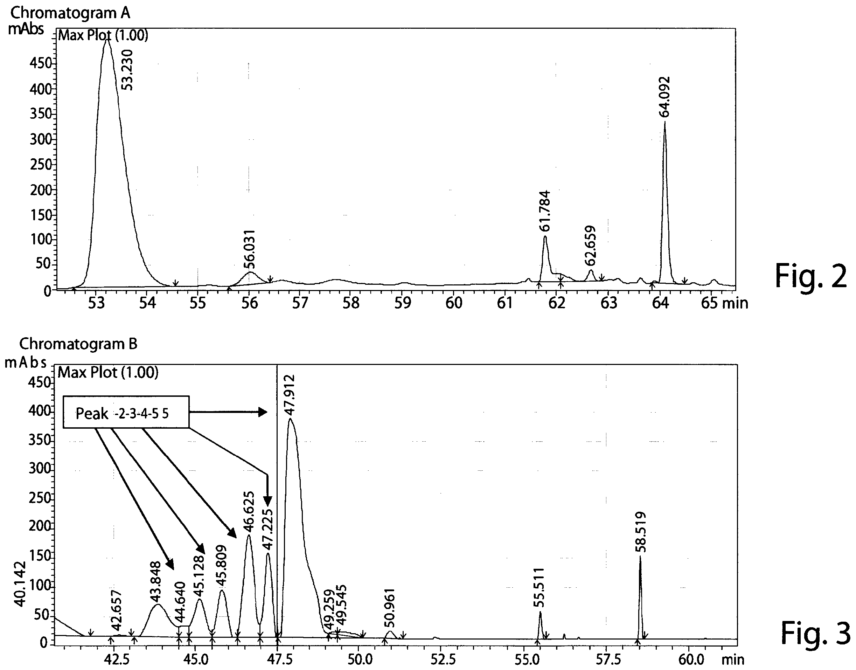

Figure 2 (chromatogram A) shows the chromatogram (OleoRub A), obtained for the OLEO-RUB ESS sample. The peak RT 53.23 is probably representative of the polyphenolic fraction yielded by Rubus Ulmifolius to the olive oil (official pharmacopoeia) during the extraction step. In order to visualise the peak of interest, the sample to be subjected to analysis was prepared with a higher concentration of extract (10 g instead of 2 g) leaving the analytical conditions unaltered (Figure 3, chromatogram B). As can be noted from chromatogram B, the analysis performed with a higher concentration of extract allowed 5 distinct peaks to be identified, relatively well resolved. It is also to be noted that the higher concentration of extract along with the use for the analysis of a new 25 cm x 4.6 mm diameter 5 micron Waters Symmetry column determined a general shift in retention times of about 5 points. Below is the method used for the analysis relative to chromatogram B (10 g extract) which as previously mentioned is the same one already applied to the first step of the study for the visualisation of chromatogram A still in relation to the OLEO-RUB.ESS sample:

- Instrument used: HPLC Shimadzu LC 2010, pump LC-10ADvp;
- Column: Waters Symmetry RP18 - 25 cm x 4.6 millimetres diameter 5 micron;
- Mobile phase: Water:Acetic acid 97:3 (solvent A) - Acetonitrile:Methanol 50:50 (solvent B);
- Duration of chromatographic run: 67 minutes Room T;
- Flow 1 ml/minute.

**Treating the sample:**

10 grams of OLEO-RUB.ESS were initially weighed, to which 50 ml of Hexane were subsequently added. The sample was then placed on a magnetic stirrer for 10 minutes and then subjected to liquid-liquid extraction in a separating funnel performed three times with the addition of 20 ml of a Methanol:Water solution, 80:20. The extract was then taken dry to the rotavapor and rehydrated before injection in HPLC with 1 ml of methanol.

**Fresh drug - dry drug comparison**

To confirm how the drying process does not determine a reduction in the polyphenolic presence in the drug and consequently in the extract, the chromatograms obtained by screening the fresh drug and the dry drug are compared below. The sample of dry drug to be subjected to analysis was prepared following the same protocol used for the fresh drug, summarised below.

The sample was prepared by inserting 1 gram of dry drug (buds) in a glass column containing 10 ml of Methanol and subsequently subjected to grinding, operating at room temperature with Ultraturrax for 2-3 minutes.

The contents of the column are then poured into a vacuum flask equipped with a ceramic Buchner and filter for vacuum filtration, then the filtrate is inserted into a glass round-bottomed flask and placed into the rotavapor in order to completely remove the solvent operating at temperatures of no greater than 30-35°C. The sample of drug that remains in the filter is subjected two more times to the same process and the resulting filtrate is added to the round-bottomed flask containing the previous fractions. Once the solvent has been completely removed, redissolving with 1 ml of Methanol and HPLC analysis is carried out. Instrument used: HPLC Shimadzu LC 2010, pump LC-10ADvp; Column: Waters Symmetry RP18 - 25 cm x 4.6 millimetres diameter 5 micron. Duration of chromatographic run 35 minutes Room T, Mobile phase (A): Methanol:Water - 90:10; (B) Water:Methanol - 90:10.

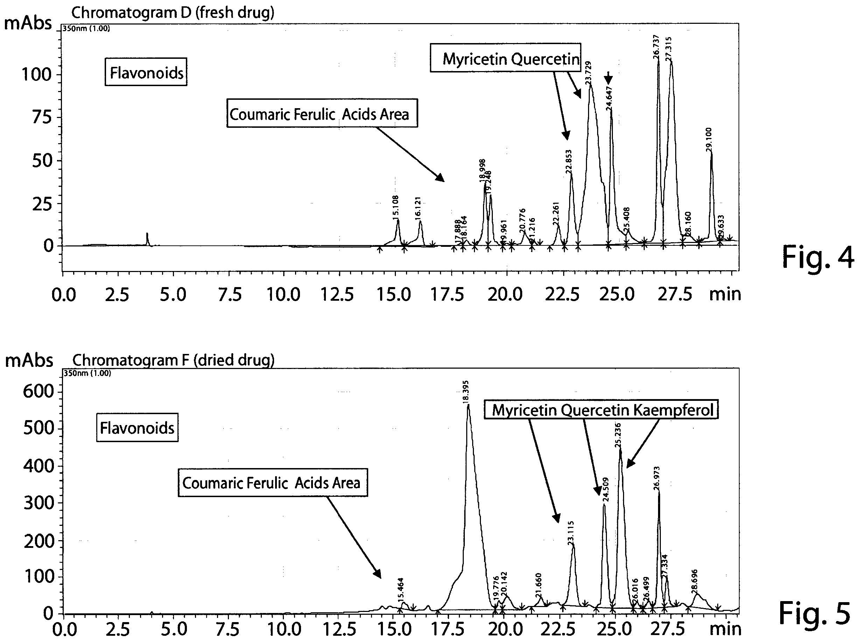

As can be noted from the chromatograms shown in the figures, the drying process does not interfere with the presence of the polyphenolic component found in the fresh drug. On the contrary, many peaks are certainly more concentrated in chromatogram F (Figure 5) (dry drug) even if this determines a slight shift in the retention times.

**Quality/quantity characterisation of OLEO-RUB.ESS**

For the quality/quantity determination of the peaks identified in the OLEO-RUB.ESS sample, as shown in chromatogram B, the sample was subjected to 5 fractionations. Each fraction was then subjected to HPLC-MS investigation in order to qualitatively identify the component responsible for the peak, which, once identified, after preparing the respective standard solution and relevant calibration line, was also quantified. The analytical conditions used for the fractionated peaks are reported as follows:

- Instrument used: HPLC Shimadzu LC 2010, pump LC-10ADvp;
- Column: Waters Symmetry RP18 - 25 cm x 4.6 millimetres diameter 5 micron;
- Mobile phase Water:Methanol, 90:10 (Solvent A) - Methanol:Water 90:10 (Solvent B);
- Duration of chromatographic run: 35 minutes Room T;

For the mass:

- Instrument used: HPLC-MS Thermo LTX XL Linear Ion Trap Mass Spectrometer;
- Column: Waters Symmetry RP18 - 25 cm x 4.6 millimetres diameter 5 micron;
- Mobile phase Water:Acetic acid 97:3 (solvent A) Acetonitrile:Methanol 50:50 (solvent B);
- Duration of chromatographic run: 67 minutes Room T.

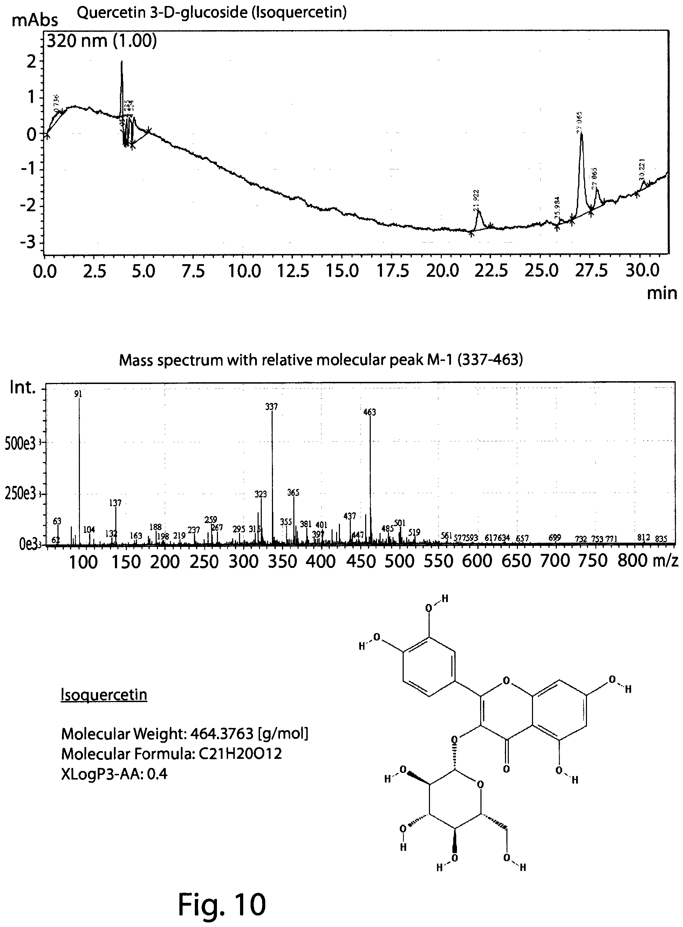

The component qualitatively identified in peak 5 (Isoquercetin) (Figure 10) was not identified since it was not possible to find the standard. However, the total area of only 400,000 along with the simultaneous presence of isoquercetin dihydrate suggests a presence of about 40% of the total i.e. concentrations that should not exceed 0.054 mg of Isoquercetin in 10 grams of OLEO-RUB.ESS.

Summary of components per 10 grams of OLEO-RUB.ESS:

| Components | | Concentration per 10 g of OLEO-RUB.ESS | |
|---|---|---|---|
| | | | |
| 1 | Caffeic acid | 5.60 mg | Exact concentration |

(continued)

| Components | | Concentration per 10 g of OLEO-RUB.ESS | |
|---|---|---|---|
| 2 | Rhamnetin | 8.94 mg | Exact concentration |
| 3 | Kaempferol | 0.57 mg | Exact concentration |
| 4 | Quercetin | 0.68 mg | Exact concentration |
| 5b | Isoquercetin | 0.054 mg | Estimated concentration |

## Preparation method

### OLEO-RUB 1 PROTOTYPE

With respect to the product described in Italian patent IT0011265649, the following components have been replaced:
Lamb/sheep fat replaced with anhydrous lanolin (official pharmacopoeia); Extra virgin olive oil replaced with virgin olive oil (official pharmacopoeia); Bees' wax replaced with cera alba/white flakes (official pharmacopoeia); An aliquot of unsaponifiables of olive oil was also added (which represent the olive oil fraction richest in polyphenolic components) in order to enrich the refined olive oil (official pharmacopoeia) and bring it closer to the characteristics of the olive oil in the patent indicated above (extra virgin olive oil).

Quantity of components per 100 grams:

| | |
|---|---|
| OLEO-RUB.ESS | 40 g |
| Anhydrous lanolin (official pharmacopoeia) | 25 g |
| Cera Alba/White flakes (official pharmacopoeia) | 8 g |
| Unsaponifiables of olive oil (official pharmacopoeia) | 4.89 g |
| Sterile water | 21 g |
| BHT (antioxidant) | 0.01 g |
| Sodium Benzoate (preservative) | 0.1 g |

30-50 g preferably 40 g Extract in Olive Oil from the official pharmacopoeia of dried Rubus buds [OLEO-RUB.ESS];
15-35 g preferably 25 g Anhydrous Lanolin (official pharmacopoeia);
6-10 g preferably 8 g cera alba/white flakes (official pharmacopoeia);
3-5 g preferably 4.89 g Unsaponifiables of olive oil (official pharmacopoeia) ;
16-26 g preferably 21 g Sterile water;
0.005-0.015 g preferably 0.01 g antioxidant (e.g. BHT);
0.05-0.015 g preferably 0.1 g preservative (e.g. Sodium Benzoate).

## Preparation:

OLEO-RUB1 prototype

The appropriate quantities of lanolin and white wax are melted at about 70°C and subsequently the extract (OLEO-RUB.ESS) and the unsaponifiables of olive oil are added. The preservative (Sodium Benzoate) and the antioxidant (BHT) are added to the fatty mass. The semi-processed product is then subjected to turbo emulsion initially operating at 2000 rpm through an emulsifying sieve (wide mesh) during this phase which lasts about 10 minutes the envisaged quantity of water is also added, first heated to about 70°C in order to prevent instantaneous solidification of the molten mass. A slower process guarantees more efficient and stable incorporation of the water in the internal phase of the emulsion. After about 15 minutes the homogenisation through turbo emulsion continues at 3000/3500 rpm through the emulsifying sieve with narrow mesh and continues for about 30 minutes. At this stage the initially fluid mass begins to gain viscosity and the process continues for another 30 minutes reducing the speed of the turbo emulsifier (1500 rpm) and activating planetary agitation. Finally, after this stage, the formulation is left under planetary agitation only and when the mass reaches a temperature of about 37°C, hence before it has reached the final viscosity, it is poured into the container that is only sealed once solidification is complete, i.e. when the temperature settles around 27°C.

*OLEO-RUB 2 Prototype*

The process is the same as the previous one, OLEO-RUB 1, except that the fraction of unsaponifiables is replaced with olive oil (official pharmacopoeia).

Composition per 100 grams:

| | |
|---|---|
| **OLEO-RUB.ESS** | **40 g** |
| **Anhydrous lanolin (official pharmacopoeia)** | **25 g** |
| **Cera Alba/White flakes (official pharmacopoeia)** | **8 g** |
| **Virgin olive oil (official pharmacopeia)** | **4.89 g** |
| **Sterile water** | **21 g** |
| **BHT (antioxidant)** | **0.01 g** |
| **Sodium Benzoate (preservative)** | **0.1 g** |

*OLEO-RUB 3 Prototype*

Unlike the previous formulations, in this prototype an emulsifying system has been used made up of Cetostearyl Alcohol and Cetomacrogol 1000 with the function of giving the formulation a creamier consistency typical of a W/O emulsion.

Composition per 100 grams:

| | |
|---|---|
| **OLEO-RUB.ESS** | **40 g** |
| **Anhydrous lanolin (official pharmacopoeia)** | **17 g** |
| **Unsaponifiables of olive oil (official pharmacopoeia)** | **4.89 g** |
| **Cetostearyl alcohol** | **5.5 g** |
| **Cetomacrogol 1000** | **2.5 g** |
| **Sterile water** | **29 g** |
| **BHT (antioxidant)** | **0.01 g** |
| **Sodium Benzoate (preservative)** | **0.1 g** |

The following table reports the quantities of Caffeic Acid, Rhamnetin, Kaempferol, Quercetin, Quercetin dihydrate and Isoquercetin expressed in mg per 100g of final product.

| | **Components** | | **mg per 100 g of product** | | |
|---|---|---|---|---|---|
| | | | **OLEO-RUB 1** | **OLEO-RUB 2** | **OLEO-RUB 3** |
| **1** | | **Caffeic acid** | **22.4** | **22.4** | **22.4** |
| **2** | | **Rhamnetin** | **35.76** | **35.76** | **35.76** |
| **3** | | **Kaempferol** | **2.28** | **2.28** | **2.28** |
| **4** | | **Quercetin** | **2.72** | **2.72** | **2.72** |
| **5a** | | **Quercetin dihydrate** | **0.324*** | **0.324*** | **0.324*** |
| **5b** | | **Isoquercetin** | **0.216*** | **0.216*** | **0.216*** |
| *estimated quantity | | | | | |

## Stability assessment of the final product

### a. *Chemical/physical and organoleptic stability*

The assessment of the chemical/physical and organoleptic stability was performed using methods commonly accepted by the scientific community for this category of products. The prototypes (OLEO-RUB 1; OLEO-RUB 2; and OLEO-RUB 3) were subjected to heating, freezing and centrifuge cycles operating as follows:

- Heating in a ventilated stove to 50°C for 1 hour,
- Stabilisation at room temperature for 1 hour
- Freezing to -21 °C for 2 hours
- Stabilisation at room temperature for 2 hours
- Centrifugation at 3000 rpm for 15 minutes
- Check after 18 hours

This protocol was applied to all three prototypes starting from 24 hours after preparation and repeating the test every 15 days for a period of 2 months date/preparation with the following outcome:

| Compliance with respect to initial pre-test characteristics after 2 months | | | |
|---|---|---|---|
| | OLEO-RUB 1 | OLEO-RUB 2 | OLEO-RUB 3 |
| Odour | compliant | compliant | compliant |
| Colour | compliant | compliant | compliant |
| Consistency/ Viscosity | compliant | compliant | Slightly more fluid |
| Homogeneity | Superficial waxing | Superficial waxing | compliant |
| Surfacing/ coalescence | none | none | none |

### *Microbiological stability*

The investigations used to check the microbiological stability were performed on samples 24 hours after preparation up to samples at 2 months. The protocol envisages the use of Sabouraud Dextrose Agar/Tryptic Soy Agar on which the samples are plated at different dilutions and subsequently incubated at the required temperature for a period comprised between 48 hours and 3 weeks in the event of mould and yeast.

| Microbiological check 2 months after preparation | | | |
|---|---|---|---|
| | OLEO-RUB 1 | OLEO-RUB 2 | OLEO-RUB 3 |
| Microorganisms | absent | absent | absent |
| Yeast/mould | absent | absent | absent |

As expected, apart from the presence of a suitable preservative, the almost total absence of free water probably creates absolutely unfavourable conditions for microbial proliferation, which could be interesting considering, along with special types of packaging (Airless), the possibility of not adding any preservative.

### b. *Multiresidue pesticide analysis*

The analyses on a sample of dry drug were performed for the purpose of verifying the presence of residues deriving from pesticides; in particular the investigation performed through GC-ITMS; HPLC-DAD focused on searching for: Alalcor, Aldrin + Eldrin, Azinphos-methyl, Carbon disulphide, Chlorpyrifos, Chlorpyrifos methyl, DDT, Dichlorvos, Dimethoate, Disulfoton, Dithiocarbamates, Endosulfan (a and β), Endrin, Heptachlor + epoxide, Hexachlorobenzene, Fenchlorphos, Fenofos, Phosalone, a-, β-, HCH, y, 5, and E-HCH, Lindane, Malathion, Methoxychlor, Parathion, Parathion-methyl, Perthane, Pirimiphos-methyl.

**Outcome of multiresidue pesticide analysis < 0.01 ppm**

c. *Heavy metal analysis*

The heavy metal concentration was measured on a sample of dry drug using optical ICP-AES (Inductively Coupled Plasma-Atomic Emission Spectrometry) after mineralisation with aqua regia in a microwave oven. This analysis was used to assess the presence of:
Antimony, Arsenic, Boron, Cadmium, Cobalt, Total Chromium, Mercury, Nickel, Lead, Selenium, Tin, Strontium, Thallium, Tellurium, Titanium, Vanadium, Zinc.

**Outcome of heavy metal analysis < 0.5 ppm**

With regard to the characterisation analyses, we can state that the extract presents a discrete concentration of substances of a polyphenolic nature such as caffeic acid, Kaempferol and Quercetin with its derivatives, which are widely described in literature in terms of their excellent antioxidant properties largely exploited both in the sector of dietary supplements and in the dermatological sector.

**PART 2: Efficacy and toxicity assessment**

The research activity aimed to provide a preliminary assessment of the efficacy and tolerability of the Cream according to the invention, MVT Skin Care, after topical administration. In particular, the intention was to test the ability of the preparation being examined to promote tissue regeneration of the cutis after second grade burns. Furthermore, the study set out to provide a general assessment of the state of health of laboratory animals subjected to acute and chronic treatment with MVT Skin Care cream and to check the presence of any alterations to the functionalities or the structure of the liver and kidneys.

**1) EXPERIMENTAL PROTOCOL**

**2.1. Efficacy study - Macroscopic assessment of the healing process**

**2.1.1. Animals**

Male Sprague-Dawley rats (Charles River, Calco, Como, Italy) were used for the study, kept at a temperature of 22 ± 2C°, relative humidity 55 ± 10%, with a light/dark cycle of 12 hours, food and water *ad libitum.* The experimental activity began at least 7 days after the animals arrived in the laboratory at the stalling facility, so as to enable them to suitably adapt to the new environmental conditions.

**2.1.2. Experimental model**

On the day of the intervention the rats were anaesthetised by intraperitoneal administration of Equithesin. After the laboratory animal had reached a deep state of unconsciousness, the animal's back was shaved (anterior part of the back), which was then disinfected and exposed to a heat source in one point. The heat source comprised a small circular metal plate (diameter 1.5 cm) heated to a temperature of 80°C. The plate was positioned on the animal's cutis for 15 seconds. For the purpose of standardising the method, the plate was rested on the animal's cutis without applying any pressure, apart from that connected with the weight of the plate itself (100 g). Immediately after contact with the hot plate, the cutis was cooled with physiological solution at 4°C for 10 seconds.

**2.1.3. Topical treatment and macroscopic assessment of the healing process**

Topical administration was performed twice a day for a period of 15 days. The administration consisted of placing a sufficient quantity of the compound being examined to cover the whole area of skin previously exposed to the heat source. The wounds were washed with sterile physiological solution before every dressing.
The experimental protocol comprised the following experimental groups:

1) Rats exposed to the heat source and not treated (whose wound was only washed with sterile physiological solution)
2) Rats exposed to the heat source treated with the commercial product Connettivina
3) Rats exposed to the heat source treated with the Cream MVT Skin Care (OLEO-RUB 2 formulation)

Each experimental group consisted of 15 rats.

The macroscopic assessment of the healing process of the wound was performed on the 1st, 3rd, 5th, 7th, 13th and 15th day. The wounds were photographed with a digital camera (Coolpix, Nikon, USA) and analysed using image analysis software (Digimizer 4.2, MedCalc Software, Belgium) to assess the healing status. In particular, the area of the wound was calculated which, due to the presence of scabs or clear redness, appeared to have an altered macroscopic structure.

The healing speed of the burn was expressed as the wound contraction ratio (WCR%):

$$WCR\% = \frac{A_0 - At}{A_0} \times 100\%$$

Where $A_0$ and At refer to the initial area of the wound and the area at time t, respectively.

Statistical analysis of the experimental data was performed using the Two-way test ANOVA for repeated tests, followed by the Bonferroni test for the internal comparison of the groups.

The liver and kidneys of the laboratory animals were taken out and placed in paraformaldehyde at 4% for histological analysis if required.

## 2. 2. Systemic toxicity studies

### 2.2.1 Design and experimental groups

In order to assess whether during the course of the entire treatment undesired phenomena could be highlighted in the experimental groups subject to treatment with the preparation being examined, some parameters were evaluated for the purpose of experimentally assessing the toxicity of the product. The first datum evaluated was whether during treatment the death of any individuals or the presence of clear behavioural modifications attributable to an altered state of health could be observed.

For the purpose of highlighting a possible toxic or harmful effect of the preparation being examined after acute and chronic topical administration (15 days, twice a day), the presence of any morphological modifications in the liver and kidney tissue was also assessed through autoptic and histological tests. Finally, some urine and blood tests were performed which could indicate compromised liver or kidney function.

In particular, the possible damage to liver function was assessed by analysing the plasma levels of the following markers:

✓ Alanine aminotransferase (ALT)
✓ Aspartate aminotransferase (AST)
✓ Alkaline phosphatase (ALP)

To assess the efficacy of the cicatrisation phenomena, but also the possible worsening thereof following treatment with the preparation, some hematic parameters were measured, such as:

✓ Albumin (ALB)
✓ Prothrombin time (PT)
✓ Fibrinogen

To assess possible damage to kidney function the following markers were measured:

✓ Creatinine
✓ Azotaemia
✓ Chemical-physical urinalysis: specific weight, pH, presence/absence (on ordinal scale)
of: protein, blood, leukocytes, bilirubin, glucose, ketones.

The experimental groups provided were:

1) Rats exposed to the heat source and not treated (whose wound was only washed with sterile physiological solution)
2) Rats exposed to the heat source and treated with the cream MVT Skin Care

The experiments were performed on 6 animals per experimental group.

### 2.2.2. Experimental procedure

#### 2.2.2. 1. Chronic treatment

After the burn, the rats were subjected to chronic topical treatment (15 days, administration twice a day) as previously indicated. Two hours after the last topical administration with MVT Skin Care Cream and with physiological solution only, a urine sample was taken and the laboratory animal was anaesthetised. Once the laboratory animals had reached a state of deep unconsciousness induced by the intraperitoneal administration of equithesin, an intracardiac blood sample was taken and macroscopic analysis of the organs present in the abdominal cavity was performed.
Finally, the liver and kidneys were removed, whose macroscopic structure was observed, to then be quickly fixed in 4% paraformaldehyde.

#### 2.2.2.2. Acute treatment

The laboratory animals were anaesthetised in order to be able to perform the burn protocol previously described. Two hours after the application of the hot plate, topical administration of MVT Skin Care Cream or physiological solution alone was performed. Two hours after administration, the urine sample was taken and the laboratory animal was anaesthetised. Once the laboratory animals had reached a state of deep unconsciousness, an intracardiac blood sample was taken and macroscopic analysis of the organs present in the abdominal cavity was performed. Finally, the liver and kidneys were removed, whose macroscopic structure was observed, to then be quickly fixed in 4% paraformaldehyde.
With reference to the kidney parameters, equithesin was used as an anaesthetic, whereas the hepatic parameters were evaluated in animals subjected to anaesthetic/analgesic with morphine (5 mg/kg SC) and medetomidine (0.1 mg/kg, IM).
The use of different types of anaesthetic/analgesic was necessary due to the complaint that repeated administration of equithesin caused an excessive alteration of the hepatic function parameters. Consequently, it was preferred to repeat the experiment using an analgesic/anaesthetic procedure with a lower impact on hepatic function.

### 2.2.3. Analysis procedure

#### 2.2.3.1. Blood and urine analyses

The biochemical/clinical analyses (ALB, AST, ALT, ALP, Urea and Creatinine) were performed using an automatic biochemical analyser (BS-120 Chemistry analyzer, Mindray Medical International Limited, Milan, Italy) and specific reactants bought from Mindray. The chemical/physical urine test was conducted using a reflectance photometer (UA Vetlab analyzer, Idexx Laboratories Inc., Novara, Italy) and Idexx UA stripes reactants, while the Prothrombin time and Fibrinogen were measured using a coagulometer (ACL 7000, IL Instrumentation Laboratory, Milan, Italy) and IL Instrumentation Laboratory reactants. The fibrinogen was measured using the Clauss method.

#### 2.2.3.1. Histological analyses

The kidney and liver tissue fixed with 4% paraformaldehyde was dissected into slices 15 $\mu$m thick using a cryostat (CM-3050, Leica, Germany). The slices of tissue were assembled onto a slide and coloured using the haematoxylin and eosin colouring method. The microscopic structure was visualised using a brightfield microscope (BX-60, Olympus, Italy).

### 3) RESULTS

The invention is described as a therapeutic dermatological product for topical use, very effective as a coadjuvant in therapy for burns and skin pathologies in general, particularly of the inflammatory type, control and attenuation of wounds and, considering its origin, it can be considered a traditional plant medication. The demonstration of the essential requirements (efficacy and safety) was obtained with a careful clinical assessment based on pre-existing clinical data and *ad hoc* clinical investigations. It can be stated that in the clinical assessment of the product, the essential safety and intrinsic performance requirements thereof were considered. The product, which exclusively comprises vegetable extracts from medicinal plants, has enjoyed a sufficiently long and constant tradition and a very long period of use for over 200 years and up to now its valid efficacy has been demonstrated based on long-term use and experience. The invention, which has been shown to be innocuous under normal conditions of use, has astringent, antiphlogistic, antiseptic (meaning a protective barrier to entry from the outside and against the growth of pathogenic germs), haemostatic and soothing actions in a single solution. The extract is very effective in the treatment of burns and, in general, all cutaneous-mucosal pathologies that require the reactivation of epithelial neoformation processes: burns, bruises, grazes, abrasions, rhagades, ulcers, radiodermatitis, bedsores, torpid sores, traumatic and surgical wounds, eczema, chilblains, dyshidrosis, itching, allergic rash, insect bites, herpetic and erythematous lesions, dermatitis, dermatosis, folliculitis, furunculosis, sunburn, post-laser erythema, nappy rash, vulvitis and vaginitis, acne rosacea and vulgaris, and as a coadjuvant in the cicatrisation of surgical wounds etc. The product's marked beneficial coadjuvant-therapeutic, effective and fast action is performed on the surfaces of the skin and mucosa through the formation of a protective cuticle against chemical, bacterial, mechanical and irritative agents in general, probably due to the precipitation and transformation of the tissue proteins. This leads to reduced tissue secretion and removal of water from the inflamed tissues. In burns, in particular, it soothes pain, reduces losses of serum, protects the wound against bacterial invasion, promotes excellent tissue cicatrisation, almost completely reducing scarring retraction with complete restitution of the newly formed tissues, providing excellent elasticity of the skin and mucosa. Repeated application of the product in the pathologies indicated above has never highlighted, either before or over the last 20 years of use, any immediate and/or delayed local or systemic side effect and neither have any cases of individual hypersensitivity or adverse reactions to the product occurred.

3.1. *Efficacy study - Macroscopic assessment of the healing process*

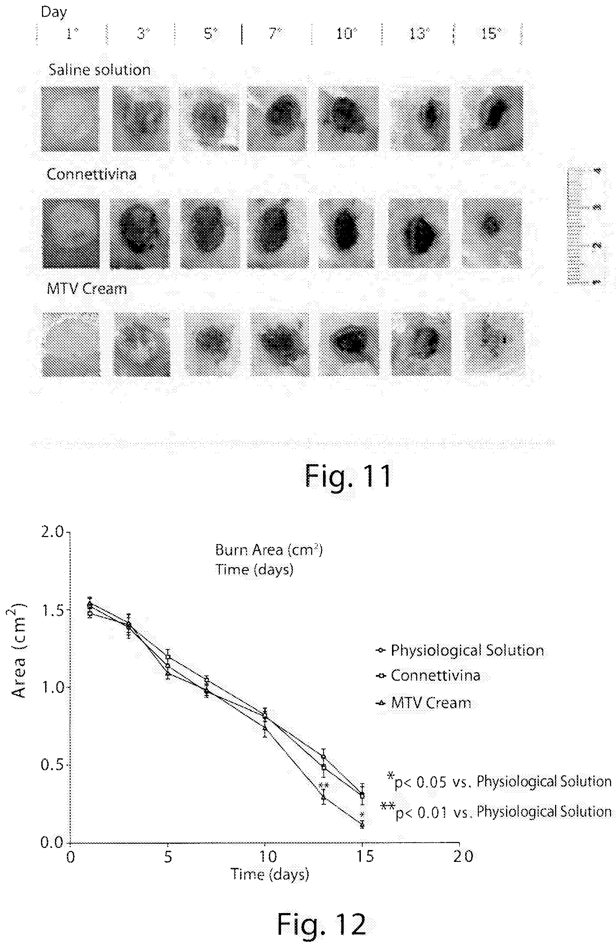

The application of the hot plate produced a clear emergence of the blister that characterises second degree burns (Figure 11). The normal evolution of the wound led to the formation of a scab that lasted a number of days (Figure 11). After the scab had fallen off, generally a modest sized residual lesion was noted on the cutaneous tissue (Figure 11). The effect of the different topical treatments (physiological solution, Connettivina, MVT oleo rub 2) on the area of skin affected by the burn highlighted that in all the experimental groups there was a significant reduction in the area of the wound as time passed but that there were significant differences between the values of the areas of the wounds treated with the products mentioned above, which enabled us to draw the following conclusions: macroscopic analysis of the wound indicates that MVT Skin Care Cream accelerates the healing time of a $2^{nd}$ degree burn lesion.

**EXAMPLE 2:** *Comparison between phytochemical characteristics of the extract according to the present invention and that obtained according to Italian patent IT 0001265649 and assessment of the efficacy of the resulting ointments in the treatment of cutaneous and mucosal lesions.*

The comparison between the extracts obtained through the procedure described in document IT 0001265649 (ITCA930003) (OLEO-RUB sample) and that obtained through the method according to the present patent application (OLEO-RUB.ESS sample) highlights how the extraction procedure according to the present invention causes an undoubtedly higher extractive capacity in reference to active phytocomplexes of a polyphenolic nature, i.e. the main substances responsible for the therapeutic activity found in the resulting ointment.

*OLEO RUB sample* - obtained by inserting 25 grams of Rubus Ulmifolius buds in 75 grams of refined olive oil (official pharmacopoeia specifications) previously heated to 150°C. The buds are left in oil for 2 minutes monitoring the temperature which undergoes an initial reduction of about 25°C (hence the temperature reaches 125°C) then returning to 142°C after the 2 minutes, the time in which the extraction process finishes. The oil and buds are moved away from the heat source and the oil is filtered and appropriately preserved.

*OLEO-RUB.ESS sample* obtained by inserting 7.5 grams (7.5 grams of dried buds which correspond to 25 grams of fresh buds) of Rubus Ulmifolius buds, previously dried in a ventilated stove at 40°C for 24 hours, in 92.5 grams of refined olive oil (official pharmacopeia specifications). The extraction was performed leaving the drug in contact with the oil for 240 hours (10 days) at room temperature ($\pm$ 25°C). After 10 days the sample was filtered and appropriately stored for suitable analyses.

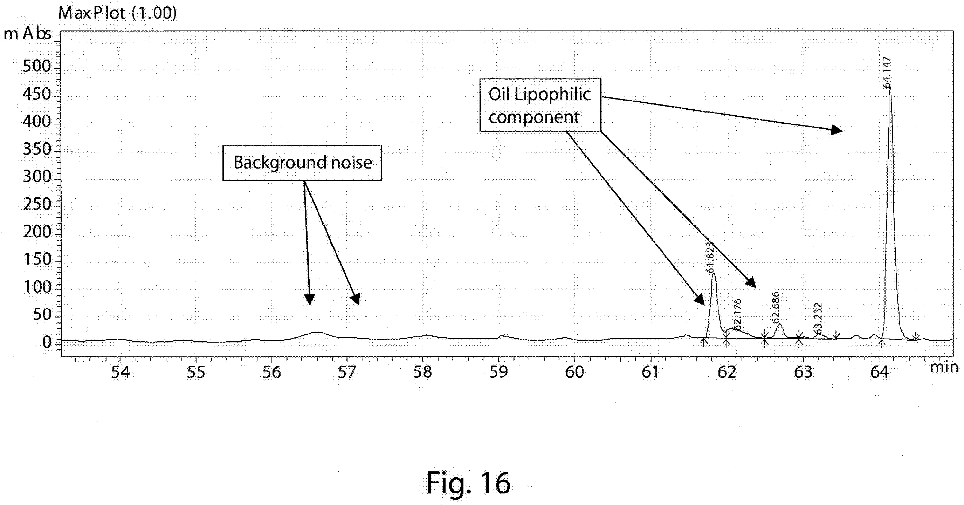

Figure 16 shows the results of the HPLC analysis conducted on the extract obtained hot according to the prior art (*OLEO-RUB sample*).

From the analysis of the OLEO-RUB sample, obtained using medicinal oil and extraction according to the method of the prior art, i.e. heating the oil to 150°C and then adding an appropriate quantity of buds, some differences are highlighted with respect to the blank. In the first place, a certain background noise can be heard in the main area of interest (52-57) which indicates the presence, although infinitesimal, of something that derives from the bud which, probably due to the reduced extraction time and also the absolute quantities used (also in the analysis), does not lead to a well resolved peak. Therefore, it was not possible to instrumentally detect which active substances are potentially present in the extract or their concentration. In the second place, further to a small shift in retention times, two small peaks can be seen at 62.176 and 63.232 which were not present in the blank. For these peaks it is justifiable to consider that they derive from a fractionation and/or modification of the responsible component(s) respectively of this signal at retention time 61.823 and 62.686 hence indicating that these peaks do not derive from the bud but from components already present in the oil.

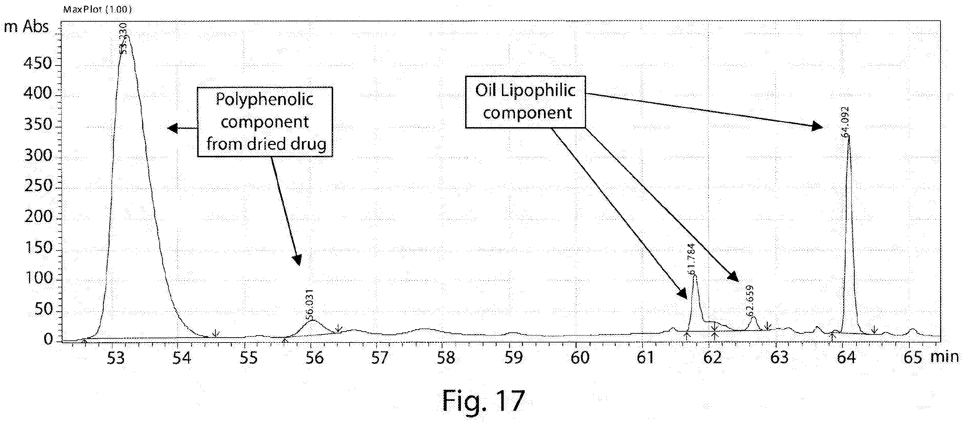

Figure 17 shows the results of the HPLC analysis conducted on the extract obtained according to the present invention (**OLEO-RUB ESS sample**).

Analysis of the OLEO-RUB ESS sample shows, contrary to the previous sample, a clear peak in the range 53-54 and also around 56 demonstrating that with this extraction technique a significant quantity of the component of a polyphenolic nature deriving from the drug is present, which in the case in question is represented by Rubus Ulmifolius dry bud.

The identity of the substances to which the first peak can be attributed, i.e. the largest one at time 53.23, was detected using an appropriate fractionation process, along with the use of a standard. Finally, by analysing the peaks relative to the oil component in the OLEO-RUB ESS sample, fractionations and/or modifications of the peaks are not visualised at 62.176 and 63.232 and in any case the "noise present" may only be attributable to their presence in a very reduced quantity. This consideration could be correlated to the absence of heating which could for example promote bland oxidation phenomena with the consequent modification of the retention times of the original species with the formation of peaks around 62.176 and 63.232.

Following the fractionation and identification of the component present in the first peak of the extract according to the present invention, it was possible to identify that the main components were Caffeic Acid, Rhamnetin, Kaempferol and Quercetin.

Unlike the description contained in the document ITCA930003, wherein the peaks relative to the polyphenolic component as above are difficult to highlight, the extraction process according to the present invention allows the extraction of a much higher significant quantity of polyphenolic component with respect to the traces only just detectable in the extract obtained according to the prior art. Hence, the ointment according to the present invention will be characterised by an undoubtedly higher concentration of active phytocomplex in comparison with the product indicated in document ITCA930003. The active species characterised and that certainly represent important exponents of the active phytocomplex present in the extract are, for example:
0.3% (w/w) of Caffeic Acid or however at least 0.2% (w/w); 0.41% (w/w) of Rhamnetin or however at least 0.3% (w/w); 0.35% (w/w) of Quercetin or however at least 0.3% (w/w) and 0.25% (w/w) of Kaempferol or however at least 0.2% (w/w).

The extract according to the present invention is more effective in the treatment of cutaneous and mucosal pathologies in comparison with the extract described in Italian patent IT0001265649. In particular, for the same cutaneous or mucosal lesion and administration, the extract according to the present invention determines a more marked action promoting cutaneous regeneration and quicker healing in comparison with the extract prepared according to the prior art.

**Claims**

1. Oily extract of plants of *Rubus* species, said extract being obtained by cold oil extraction starting from dry plant samples and comprising the polyphenolic phytocomplex of said plants wherein said polyphenolic phytocomplex comprises Caffeic Acid, Rhamnetin, Quercetin, Kaempferol and wherein said dry plant samples are dried buds.

2. Extract according to claim 1, wherein said polyphenolic phytocomplex comprises Caffeic Acid at the concentration of at least 0.2% (w/w), Rhamnetin at the concentration of at least 0.3% (w/w), Quercetin at the concentration of at least 0.3% (w/w) and Kaempferol at the concentration of at least 0.2% (w/w), wherein said percentages are weight percentages of each compound with respect to the weight of the extract.

3. Extract according to any one of claims 1-2, wherein the *Rubus* species is chosen from the group consisting of *Rubus Ulmifolius, Rubus Idaeus, Rubus caesius, Rubus saxatillis,* from the group *Rubus Ulmifolius* preferably *Rubus ulmifolius frutticosus schott.*

4. Extract according to any one of the preceding claims wherein said oil is chosen from the group of vegetable oils consisting of almond, jojoba, sunflower, virgin olive oil, extra virgin olive oil, preferably virgin olive and extra virgin olive oils.

5. Pharmaceutical composition comprising or consisting of an extract in plant oil of the *Rubus* species as defined in any one of claims 1 to 4, as the active ingredient, along with one or more excipients and/or pharmaceutically acceptable coadjuvants.

6. Pharmaceutical composition according to claim 5, wherein said pharmaceutical composition is for topical use.

7. Process for the preparation of an oily extract of plants of *Rubus* species, said method being **characterised in that**

the extraction takes place through incubation in oil of dried parts of plants of the *Rubus* species and at a temperature that varies from 18 to 32°C, preferably from 22 to 28°C, more preferably at 25°C, for an incubation time of 5-15 days, wherein the dried parts of plants are dried buds.

8. Process according to claim 7, wherein the *Rubus* species are chosen from the group consisting of *Rubus Ulmifolius, Rubus Idaeus, Rubus caesius, Rubus saxatillis,* from the group *Rubus Ulmifolius* preferably *Rubus ulmifolius frutticosus schott.*

9. Process according to any one of claims 7 to 8, wherein the oil is chosen from the group that consists of almond, jojoba, sunflower, virgin olive oil, extra virgin olive oil, preferably virgin olive and extra virgin olive oils.

10. Process according to any one of claims 7 to 9, wherein the plants of the *Rubus* species are harvested in the spring-summer seasons.

11. Extract according to any one of claims 1 to 4 or composition according to any one of claims 5-6 for use in the treatment of skin and mucosal disorders or pathologies.

12. Extract according to any one of claims 1 to 4 or composition according to any one of claims 5-6 for use according to claim 11, wherein said skin or mucosal disorders or pathologies are chosen from the group consisting of burns, grazes, abrasions, rhagades, varicose ulcers, radiodermatitis, bedsores, torpid sores, light haemorrhages, nose bleeds, traumatic wounds, erythrodermic states, eczema, chilblains, dyshidrosis, itching, diabetic itching, insect bites, herpetic and erythematous lesions, dermatitis, like atopic dermatitis, psoriasis, dermatosis, acne rosacea and vulgaris, folliculitis and furunculosis, post-laser erythema, nappy rash and sunrash, mouth ulcers, Lichen Planus, preferably burns and as a coadjuvant in the alteration of surgical wounds.

13. Cosmetic composition comprising the extract as defined in any one of claims 1-4.

14. Use of the extract as defined in any one of claims 1-4 or of the cosmetic composition defined in claim 12, as a skin cosmetic, for example, antiageing, antioxidant or against skin imperfections such as, for example, stains, scars, cellulite and stretch marks.


**Patentansprüche**

1. Öliger Extrakt von Pflanzen der *Rubus*-Spezies, wobei der Extrakt durch kalte Ölextraktion, ausgehend von trockenen Pflanzenproben, erhalten wird und den polyphenolischen Phytokomplex der Pflanzen umfasst, wobei der polyphenolische Phytokomplex Kaffeesäure, Rhamnetin, Quercetin, Kämpferol umfasst und wobei die trockenen Pflanzenproben getrocknete Knospen sind.

2. Extrakt nach Anspruch 1, wobei der polyphenolische Phytokomplex Kaffeesäure bei der Konzentration von mindestens 0,2 Gew.-%, Rhamnetin bei der Konzentration von mindestens 0,3 Gew.-%, Quercetin bei der Konzentration von mindestens 0,3 Gew.-% und Kämpferol bei der Konzentration von mindestens 0,2 Gew.-% umfasst, wobei die Prozentsätze Gewichtsprozentsätze jeder Verbindung bezogen auf das Gewicht des Extrakts sind.

3. Extrakt nach einem der Ansprüche 1-2, wobei die *Rubus*-Spezies ausgewählt ist aus der Gruppe, bestehend aus *Rubus Ulmifolius, Rubus Idaeus, Rubus caesius, Rubus saxatillis,* aus der Gruppe *Rubus Ulmifolius* vorzugsweise *Rubus ulmifolius fruticosus schott.*

4. Extrakt nach einem der vorstehenden Ansprüche, wobei das Öl ausgewählt ist aus der Gruppe von pflanzlichen Ölen, bestehend aus Mandel-, Jojoba-, Sonnenblumen-, nativem Olivenöl, extra nativem Olivenöl, vorzugsweise nativem Olivenöl und extra nativem Olivenöl.

5. Pharmazeutische Zusammensetzung, umfassend einen oder bestehend aus einem Extrakt der *Rubus*-Spezies in Pflanzenöl nach einem der Ansprüche 1 bis 4, als Wirkstoff, zusammen mit einem oder mehreren Hilfsstoffen und/oder pharmazeutisch annehmbaren Coadjuvanzien.

6. Pharmazeutische Zusammensetzung nach Anspruch 5, wobei die pharmazeutische Zusammensetzung zur topischen Verwendung ist.

7. Prozess zur Zubereitung eines öligen Extrakts von Pflanzen der *Rubus*-Spezies, wobei das Verfahren **dadurch gekennzeichnet ist, dass** die Extraktion durch Inkubation von getrockneten Pflanzenteilen der *Rubus*-Spezies in Öl und bei einer Temperatur, die von 18 bis 32 °C, vorzugsweise von 22 bis 28 °C variiert, bevorzugter bei 25 °C stattfindet, für eine Inkubationszeit von 5-15 Tagen, wobei die getrockneten Teile der Pflanzen getrocknete Knospen sind.

8. Prozess nach Anspruch 7, wobei die *Rubus*-Spezies ausgewählt sind aus der Gruppe, bestehend aus *Rubus Ulmifolius, Rubus Idaeus, Rubus caesius, Rubus saxatillis,* aus der Gruppe *Rubus Ulmifolius* vorzugsweise *Rubus ulmifolius fruticosus schott.*

9. Prozess nach einem der Ansprüche 7 bis 8, wobei das Öl ausgewählt ist aus der Gruppe, die aus Mandel-, Jojoba-, Sonnenblumen-, nativem Olivenöl, extra nativem Olivenöl, vorzugsweise nativem Olivenöl und extra nativem Olivenöl besteht.

10. Prozess nach einem der Ansprüche 7 bis 9, wobei die Pflanzen der *Rubus*-Spezies in den Jahreszeiten Frühling-Sommer geerntet werden.

11. Extrakt nach einem der Ansprüche 1 bis 4 oder Zusammensetzung nach einem der Ansprüche 5-6 zur Verwendung bei der Behandlung von Haut- und Schleimhautstörungen oder -pathologien.

12. Extrakt nach einem der Ansprüche 1 bis 4 oder Zusammensetzung nach einem der Ansprüche 5-6 zur Verwendung nach Anspruch 11, wobei die Haut- oder Schleimhautstörungen oder -pathologien ausgewählt sind aus der Gruppe, bestehend aus Verbrennungen, Schrammen, Schürfwunden, Rhagaden, varikösen Geschwüren, Strahlendermatitis, Druckgeschwüren, torpiden Schmerzen, leichten Hämorrhagien, Nasenblutungen, traumatischen Wunden, erythrodermischen Zuständen, Ekzem, Frostbeulen, Dyshidrose, Juckreiz, diabetischem Juckreiz, Insektenbissen, herpetischen und erythematösen Läsionen, Dermatitis wie atopischer Dermatitis, Psoriasis, Dermatose, Akne rosacea und vulgaris, Follikulitis und Furunkulose, Post-Laser-Erythem, Windelausschlag und Sonnenausschlag, Mundgeschwüren, Lichen Planus, vorzugsweise Verbrennungen, und als ein Coadjuvans in der Alteration chirurgischer Wunden.

13. Kosmetische Zusammensetzung, umfassend den Extrakt nach einem der Ansprüche 1-4.

14. Verwendung des Extrakts nach einem der Ansprüche 1-4, oder der kosmetischen Zusammensetzung nach Anspruch 12, als ein Hautkosmetikum, zum Beispiel Antialterung, Antioxidationsmittel oder gegen Hautimperfektionen wie zum Beispiel Verfärbungen, Narben, Cellulite und Dehnungsstreifen.


## Revendications

1. Extrait huileux de plantes de l'espèce *Rubus,* ledit extrait étant obtenu par extraction à l'huile froide en partant d'échantillons végétaux secs et comprenant le phytocomplexe polyphénolique desdites plantes, ledit phytocomplexe polyphénolique comprenant de l'Acide Caféique, de la Rhamnétine, de la Quercétine, du Kaempférol et dans lequel lesdits échantillons végétaux secs sont des bourgeons séchés.

2. Extrait selon la revendication 1, dans lequel ledit phytocomplexe polyphénolique comprend de l'Acide Caféique à la concentration d'au moins 0,2 % (poids/poids), de la Rhamnétine à la concentration d'au moins 0,3 % (p/p), de la Quercétine à la concentration d'au moins 0,3 % (poids/poids) et du Kaempférol à la concentration d'au moins 0,2 % (poids/poids), lesdits pourcentages étant des pourcentages en poids de chaque composé par rapport au poids de l'extrait.

3. Extrait selon l'une quelconque des revendications 1 et 2, dans lequel l'espèce *Rubus* est choisie parmi le groupe comprenant *Rubus Ulmifolius, Rubus Idaeus, Rubus caesius, Rubus saxatillis,* parmi le groupe *Rubus Ulmifolius* de préférence *Rubus ulmifolius fruticosus schott.*

4. Extrait selon l'une quelconque des revendications précédentes dans lequel ladite huile est choisie parmi le groupe d'huiles végétales constitué d'huiles d'amande, de jojoba, de tournesol, d'huile d'olive vierge, d'huile d'olive extra vierge, de préférence d'huiles d'olive vierge et d'olive extra vierge.

**5.** Composition pharmaceutique comprenant ou constitué d'un extrait dans de l'huile végétale de l'espèce *Rubus* tel que défini dans l'une quelconque des revendications 1 à 4, en tant qu'ingrédient actif, ensemble avec un ou plusieurs excipients et/ou coadjuvants pharmaceutiquement acceptables.

**6.** Composition pharmaceutique selon la revendication 5, dans laquelle ladite composition pharmaceutique est destinée à un usage topique.

**7.** Procédé de préparation d'un extrait huileux de plantes de l'espèce *Rubus,* ladite méthode étant **caractérisée en ce que** l'extraction a lieu par incubation dans de l'huile de parties séchées de plantes de l'espèce *Rubus* et à une température variant de 18 à 32°C, de préférence de 22 à 28°C, de manière plus préférée à 25°C, pendant une durée d'incubation de 5 à 15 jours, dans laquelle les parties séchées des plantes sont des bourgeons séchés.

**8.** Procédé selon la revendication 7, dans lequel les espèces *Rubus* sont choisies parmi le groupe comprenant *Rubus Ulmifolius, Rubus Idaeus, Rubus caesius, Rubus saxatillis,* parmi le groupe *Rubus Ulmifolius* de préférence *Rubus ulmifolius fruticosus schott.*

**9.** Procédé selon l'une quelconque des revendications 7 à 8, dans lequel l'huile est choisie parmi le groupe constitué d'huiles d'amande, de jojoba, de tournesol, d'huile d'olive vierge, d'huile d'olive extra vierge, de préférence d'huiles d'olive vierge et d'olive extra vierge.

**10.** Procédé selon l'une quelconque des revendications 7 à 9, dans lequel les plantes de l'espèce *Rubus* sont récoltées au cours de la saison printemps-été.

**11.** Extrait selon l'une quelconque des revendications 1 à 4 ou composition selon l'une quelconque des revendications 5 et 6 pour une utilisation dans le traitement de troubles ou pathologies de la peau et de muqueuses.

**12.** Extrait selon l'une quelconque des revendications 1 à 4 ou composition selon l'une quelconque des revendications 5 à 6 pour une utilisation selon la revendication 11, dans lequel lesdits troubles ou pathologies de la peau ou de muqueuses sont choisis parmi le groupe constitué de brûlures, écorchures, abrasions , crevasses, ulcères variqueux, radiodermite, escarres, plaies torpides, hémorragies légères, saignements de nez, plaies traumatiques, états érythrodermiques, eczéma, engelures, dyshidrose, démangeaisons, démangeaisons diabétiques, piqûres d'insectes, lésions herpétiques et érythémateuses, dermatite, dermatite atopique, psoriasis, dermatose, acné rosacée et vulgaris, folliculite et furonculose, érythème post-laser, érythème fessier et éruptions dues au soleil, ulcères de la bouche, Lichen Plan, de préférence des brûlures et comme coadjuvant pour l'altération des plaies chirurgicales.

**13.** Composition cosmétique comprenant l'extrait tel que défini selon l'une quelconque des revendications 1 à 4.

**14.** Utilisation de l'extrait tel que défini selon l'une quelconque des revendications 1 à 4 ou de la composition cosmétique définie selon la revendication 12, en tant que produit cosmétique pour la peau, par exemple, antivieillissement, antioxydant ou contre des imperfections cutanées telles que, par exemple, des taches, des cicatrices, de la cellulite et des vergetures.

Fig. 1

Fig. 2

Fig. 3

Chromatogram D (fresh drug)

**Fig. 4**

Chromatogram F (dried drug)

**Fig. 5**

EP 3 007 713 B1

## Caffeic Acid

Caffeic Acid
280nm (1.00)

Caffeic Acid

## Mass spectrum with relative molecular peak M-1 (179)

Molecular Weight: 180.15742 [g/mol]
Molecular Formula: C9H8O4
XLogP3: 1.2
H-Bond Donor: 3
H-Bond Acceptor: 4

## Caffeic Acid Standard concentration 1 mg/ml ( methanol )

**area 24.654.000  rt 12.035**

MaxPlot (1.00)

# Fig. 6

## Kaempferol
### 285 nm (1.00)

Kaempferol

Mass spectrum with relative molecular peak M-1 (285)

Molecular Weight: 286.2363 [g/mol]
Molecular Formula: C15H9O6
XLogP3: 1.9
H-Bond Donor: 4
H-Bond Acceptor: 6

Kaempferol Standard concentration 1 mg/ml ( methanol )  area 48.654,000 rt 26,123
502nm (1.00)

## Fig. 7

Fig. 8

Molecular Weight: 302.2357 [g/mol]
Molecular Formula: C15H10O7
XLogP3: 1.5
H-Bond Donor: 5
H-Bond Acceptor: 7

Fig. 9

Quercetin 3-D-glucoside (Isoquercetin)

Mass spectrum with relative molecular peak M-1 (337-463)

Isoquercetin

Molecular Weight: 464.3763 [g/mol]
Molecular Formula: C21H20O12
XLogP3-AA: 0.4

## Fig. 10

Fig. 11

Fig. 12

Percentage of wound contraction (WCR%)
Time (Days)

*p< 0.05 vs. Physiological solution
**p< 0.01 vs. Physiological solution

Fig. 13

CHRONIC TREATMENT

VEIN CENTRILOBULAR

HEPATIC SINUSOIDS

HEPATOCYTES

SALINE

10X          20X

MVT

10X          20X

Fig. 14a

Fig. 14b

Fig. 15a

ACUTE TREATMENT

5X

10X

SALINE

5X

10X

MVT

Fig. 15B

Fig. 16

Fig. 17

EP 3 007 713 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IT 0001265649

- IT 0011265649

**Non-patent literature cited in the description**

- **IPEK SÜNTAR et al.** *Wound Healing Activity of Rubus Sanctus Schreber (Rosacee): Preclinical study in Animal models,* 2009

- **ESRA SARIBURUN et al.** *Journal of Food Science,* 2010, vol. 75 (4), C328-335
- **SIRIWOHARN et al.** *Journal of Food Science,* 2004, vol. 69 (4), 233-240